# EUROPEAN PATENT SPECIFICATION

(11) **EP 2 589 386 B1**
(45) Date of publication and mention of the grant of the patent: **17.06.2015**
(21) Application number: 11842457.1
(22) Date of filing: 21.12.2011
(51) Int. Cl.: A61K 31/52, A61K 45/00, A61P 27/10

(54) **METHOD FOR INHIBITING MYOPIA AND USE OF ADENYLATE CYCLASE INHIBITOR AS DRUG FOR INHIBITING MYOPIA**
VERFAHREN ZUR HEMMUNG VON KURZSICHTIGKEIT UND VERWENDUNG DES ADENYLAT-CYCLASE-HEMMERS ALS HEILMITTEL ZUR HEMMUNG VON KURZSICHTIGKEIT
PROCÉDÉ DESTINÉ À EMPÊCHER LE DÉVELOPPEMENT DE LA MYOPIE, ET UTILISATION D'UN INHIBITEUR DE L'ADÉNYLCYCLASE EN TANT QUE MÉDICAMENT DESTINÉ À EMPÊCHER LE DÉVELOPPEMENT DE LA MYOPIE

(30) Priority: 27.12.2010 CN 201010605133
(43) Date of publication of application: 08.05.2013
(73) Proprietor: School of Opthalmology & Optometry, Wenzhou Medical College, Wenzhou Zhejiang 325027 (CN)
(72) Inventor: ZHOU, Xiangtian, Wenzhou Zhejiang 325027 (CN); QU, Jia, Wenzhou Zhejiang 325027 (CN); TAO, Yijin, Wenzhou Zhejiang 325027 (CN); PAN, Miaozhen, Wenzhou Zhejiang 325027 (CN)
(74) Representative: Viering, Jentschura & Partner Patent- und Rechtsanwälte
(86) International application number: PCT/CN2011/084332
(87) International publication number: WO 2012/089053

(56) References cited:
- WO-A1-2009/139511
- WO-A2-2010/126260
- CN-A- 102 078 614
- SHELTON LILIAN ET AL: "Inhibition of Human Scleral Fibroblast Cell Attachment to Collagen Type I by TGFBIp", IOVS, vol. 50, no. 8, August 2009 (2009-08), pages 3542-3552, XP002695740, ISSN: 0146-0404
- ZHANG, WEIJUAN ET AL.: 'The effect of U73122 and SQ 22536 on the hyperplasia and TGF-p2 secretion of retinal pigment epithelium cells' CHINESE OPHTHALMIC RESEARCH vol. 28, no. 6, June 2010, pages 519 - 523

## Description

### Technical Field

The invention relates to a method of inhibiting myopia effectively.

### Background Technology

The main characteristic of human myopia is axial elongation, and the main part of axial elongation is the posterior part of eyeball. Previous studies have found that normal human eye is significantly different from myopic eye on collagen bundle structure, fiber diameter distribution, fiber configuration and so on. Now it is found that phenomenons, for example sclera becomes thinner, scleral collagen fibrils become thinner, and gradient change of the inner, middle and outer layers of fiber diameter of the sclera disappears, appear in long-time experimental myopic eyes of mammals (tree shrew, marmoset, guinea pig). These findings indicate that: in the development process of myopia, the change of sclera structure in myopic eye is a positive remodeling process.

In recent years, researches on mammal myopic models (monkey, tree shrew) showed that, in experimental myopic eyes, growth of fibroblast is inhibited, collagen synthesis is reduced and dry weight of sclera is reduced. Biomechanics studies also found that myopia resulted in a weak sclera, and caused the change of sclera stress. It is detected that: creep rate of the sclera of myopic eye increased, elasticity of the sclera increased, and failure load of the tissue decreased. Above mentioned changes mainly appeared in the posterior pole of the sclera. The biological and chemical changes of the sclera resulted in changes of physical and mechanical characteristics of the sclera, and eventually resulted in development of myopia.

In conclusion, sclera is regarded as the target tissue of occurrence and development of myopia. Hence, regulating the activation and differentiation of sclera fibroblasts may influence the occurrence and development of myopia. Kohyama T, et al. (2002) and Liu X, et al. (2004) found that regulating the intracellular cAMP (cyclic adenosine monophosphate) level may influence the activity of lung fibroblast. cAMP plays a role of second messenger in the reaction of inhibiting the activity of fibroblasts. Differentiations of lung and cardiac fibroblasts can be inhibited by increasing the intracellular cAMP level; conversely, activation and differentiation of lung fibroblast can be promoted by decreasing the cAMP level. However, at present there's no results about effect of cAMP on sclera fibroblast, in particular the effect of cAMP on myopia.

### Invention Content

The purpose of the invention is to find a method of inhibiting myopia by intervening experimental myopia through decreasing the the intraocular cAMP level.

In order to achieve above mentioned purposes, the invention adopted technical proposals as follows: inhibiting myopia by decreasing the intraocular cAMP level.

Usually three methods are adopted for decreasing the intraocular cAMP level, the first one is to decrease the cAMP synthesis: decreasing the cAMP level via adenylyl cyclase (cAMP synthetase) inhibitor, at present SQ22536 is most commonly used; the second one is to increase the degradation: decreasing the cAMP level by mainly increasing phosphodiesterases (for hydrolyzing cAMP); the third one is to antagonize the effect of cAMP: blocking or deceasing the effect of cAMP by antagonizing in the downstream link of cAMP effect.

The invention also provides a new drug for inhibiting myopia, namely application of the adenylyl cyclase inhibitor as a drug for inhibiting myopia.

Advantages of the invention comprise: myopia can be effectively inhibited by decreasing the intraocular cAMP level.

### Description of the Drawings

Figure 1 is a diagram of refraction differences between the experimental eye and the contralateral eye.
Figure 2 is a diagram of vitreous chamber depth differences between the experimental eye and the contralateral eye.
Figure 3 is a diagram of axial length differences between the experimental eye and the contralateral eye.

In attached figures, "differences" refers to the differences of refraction or eye axis parameters between the experimental eye and the contralateral eye; comparison between the vehicle group and the drug-treated group adopts one-way analysis of variance (ANOVA): "*" refers to P<0.05; "**" refers to P<0.01; "***" refers to P<0.001.

### Specific Implemention Way

Experimental animals were 3 weeks old, UK variety, three-color and short-haired guinea pigs. Monocular form-deprivation (FD) was performed by using a facemask, and the form-deprivation eyes were treated with adenylyl cyclase inhibitor (ACI) SQ22536 of different concentrations (for example 1uM and 100uM) by sub-conjunctival injection, so as to differently decrease intraocular cAMP level. Animals were divided into 4 groups randomly: form-deprivation control group (FD + non-injection), form-deprivation + vehicle control group (FD + vehicle) (vehicle here refers to 0.9% saline), form-deprivation + drug group (FD + 1uM SQ22536 and FD + 100uM SQ22536 group). Drugs were sub-conjunctival injected at 9:00am every day for 4 weeks. The contralateral eyes were not treated. Refraction were measured with an infrared eccentric refractometer (EIR), corneal curvature radius were measured with a keratometer., eye axis parameters such as vitreous chamber depths and axial lengths were measured with an A-scan ultrasonograph (11MHz) respectively before experiment, 2 weeks after treating, 4 weeks after treating.

After comparing measured parameters before the experiment and after the experiment, it was found that the myopic refractive error , the vitreous chamber depths and axial elongations of the form-deprivation eye of the drug-treated group were all less than the form-deprivation control group and the vehicle control group, and the comparison with the vehicle control group had statistic significance. Therefore, the formation of form-deprivation myopia of guinea pigs could be inhibited by decreasing intraocular cAMP level through sub-conjunctival injecting adenylyl cyclase inhibitor (ACI) SQ22536.

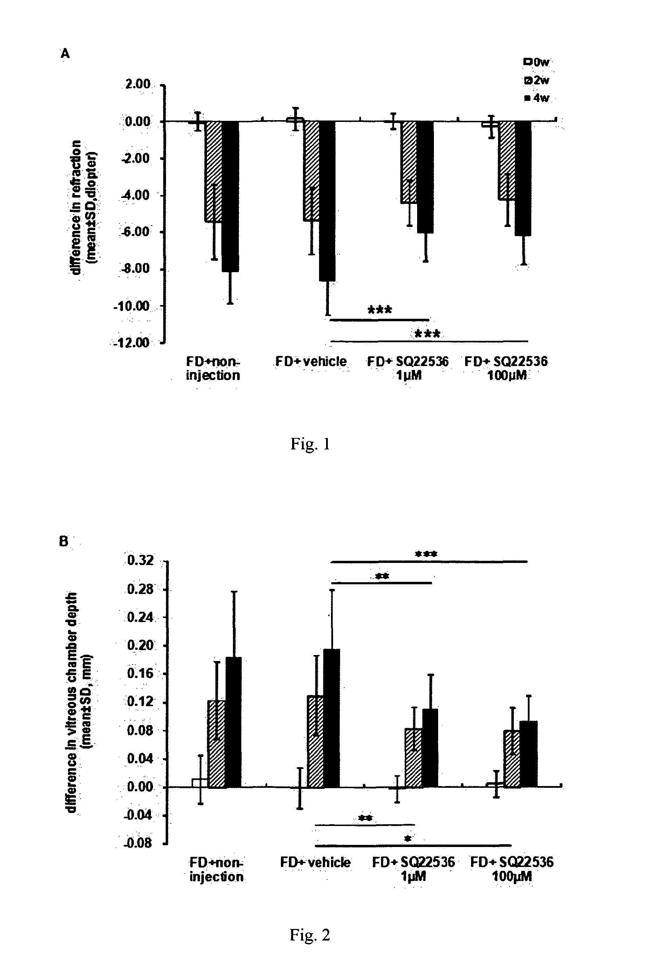

As shown in Figure 1, after 4 weeks experiment, the myopic refractive error between the form-deprivation control group and the form-deprivation and vehicle injection control group had no difference, so it showed that injection had no influence on form-deprivation. The myopic refractive error of two drug-treated groups were all less than the vehicle control group. There were a time-dependent effect and a concentration gradient effect. It showed that adenylyl cyclase inhibitor SQ22536 could inhibit the formation of form-deprivation myopia.

As shown in Figure 2, after 4 weeks experiment, the vitreous chamber elongations between the form-deprivation control group and the form-deprivation and vehicle injection control group had no difference, so it showed that injection had no influence on form-deprivation. The vitreous chamber elongations of two drug-treated groups were significantly less than the vehicle control group., There were a time-dependent effect and a concentration gradient effect., It showed that adenylyl cyclase inhibitor SQ22536 could inhibit the elongation of vitreous chamber in form-deprivation.

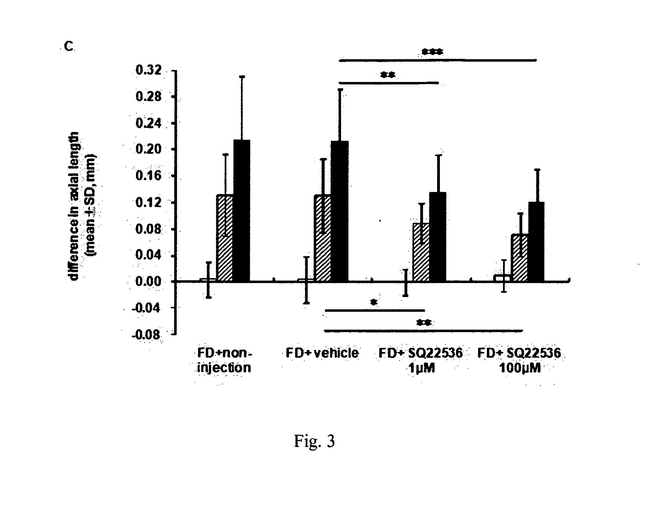

As shown in Figure 3, after 4 weeks experiment, the axial elongations between the form-deprivation control group and the form-deprivation and vehicle injection control group had no difference, so it showed that injection had no influence on form-deprivation. The axial elongations of two drug-treated groups were significantly less than the vehicle control group., There were a time-dependent effect and a concentration gradient effect., It showed that adenylyi cyclase inhibitor SQ22536 can inhibit the elongation of eye axis in form-deprivation.

Above experiment results demonstrated that myopia could be significantly inhibited by reducing the cAMP level with the adenylyl cyclase inhibitor.

## Claims

1. Composition comprising adenylyl cyclase inhibitor SQ22536 for use in an application in the treatment of a subject for inhibiting myopia.

## Patentansprüche

1. Zusammensetzung umfassend Adenylcyclase-Inhibitor SQ22536 zur Verwendung in einer Anwendung in der Behandlung eines Subjektes zur Inhibierung von Myopie.

## Revendications

1. Composition comprenant un inhibiteur d'adénylyle cyclase SQ22536 destinée à être utilisée dans une application dans le traitement d'un sujet pour inhiber la myopie.
